# FASCICULE DE BREVET EUROPEEN

(11) **EP 1 122 230 B1**
(45) Date de publication et mention de la délivrance du brevet: **25.06.2003**
(21) Numéro de dépôt: 01400172.1
(22) Date de dépôt: 19.01.2001
(51) Int. Cl.: C07C 2/36

(54) **Procédé pour la dimérisation sélective du propylène principalement en dimères ramifiés**
Verfahren zur selektiven Dimerisierung von Propen zu hauptsächlich linearen Dimeren
Process for the selective dimerisation of propylene principally to ramified dimers

(30) Priorité: 04.02.2000 FR 0001511
(43) Date de publication de la demande: 08.08.2001
(73) Titulaire: Institut Francais du Petrole, 92852 Rueil-Malmaison Cedex (FR)
(72) Inventeur: Commereuc, Dominique, 92190 Meudon (FR); Le Pennec, Dominique, 78910 Orgerus (FR); Olivier-Bourbigou, Hélène, 92500 Rueil Malmaison (FR)

(56) Documents cités:
- FR-A- 2 710 280

## Description

La présente invention concerne un procédé de dimérisation sélective du propylène principalement en dimères ramifiés. Elle concerne plus particulièrement un procédé dans lequel on met du propylène en contact avec une composition catalytique résultant de la dissolution au moins partielle d'un composé du nickel mélangé ou complexé avec au moins une phosphine tertiaire portant un groupe fonctionnel dans un milieu formé du mélange d'au moins un halogénure d'ammonium et/ou de phosphonium quaternaire avec au moins un halogénure d'aluminium et éventuellement un composé organométallique de l'aluminium.

Le brevet français FR-B-2 611 700 décrit l'utilisation de liquides à caractère ionique formés d'halogénures d'aluminium et d'halogénures d'ammonium quaternaires comme solvants de complexes organométalliques du nickel pour la catalyse de dimérisation des oléfines. L'utilisation de tels milieux non miscibles avec les hydrocarbures aliphatiques, en particulier avec les produits issus de la dimérisation des oléfines permet une meilleure utilisation des catalyseurs homogènes. Dans le brevet FR-B-2 659 871 est décrite une composition liquide à caractère ionique résultant de la mise en contact d'halogénures d'ammonium quartenaires et/ou d'halogénures de phosphonium quaternaires avec des dihalogénures d'alkylaluminium et éventuellement en outre un trihalogénure d'aluminium. Ce même brevet décrit l'utilisation de ces milieux comme solvants de complexes de métaux de transition, notamment des complexes du nickel ne contenant pas de liaison nickel-carbone, qui sont transformés en catalyseurs d'oligomérisation des oléfines. Dans la suite, ces milieux seront appelés « sels fondus » parce que liquides à température modérée.

Au cours de ces travaux, il a été montré que les catalyseurs de nickel les plus actifs et les plus stables sont obtenus dans des « sels fondus » constitués d'un équivalent molaire d'halogénure d'ammonium et/ou d'halogénure de phosphonium avec un équivalent et plus de trihalogénure d'aluminium, et éventuellement une quantité quelconque de dihalogénure d'alkyle aluminium. Cette formulation s'est révélée particulièrement intéressante parce que les complexes du nickel qui y sont dissous présentent une activité catalytique élevée.

Par ailleurs, il s'est révélé que dans les conditions décrites dans le brevet français FR-B-2 611 700 « l'effet phosphine » tel que décrit par G. Wilke et al dans Ind. Eng. Chem., 1970, 62, n°12, p. 34, et dans le brevet GB-B-1 058 680, et qui traduit l'influence des substituants portés par l'atome de phosphore sur le mode d'enchaînement des molécules de propylène lors de sa dimérisation catalytique par le nickel, disparaissait rapidement au cours du temps. Ce phénomène inexpliqué a des conséquences néfastes puisqu'il ne permet pas d'obtenir les sélectivités recherchées.

Il a été montré dans le brevet FR-B-2 710 280 que l'addition d'un hydrocarbure aromatique à un « sel fondu » permet de pallier ce défaut et conduit à des catalyseurs dont l'activité est élevée et plus stable et dont la sélectivité en isomères les plus ramifiés est importante. Cependant, l'hydrocarbure aromatique est extrait en continu dans la phase organique constituée par les produits, ce qui implique qu'il soit nécessaire de le séparer et de le recycler au réacteur.

Il a maintenant été trouvé que l'utilisation d'une phosphine tertiaire portant un groupe fonctionnel ou d'un complexe de nickel formé avec une phosphine tertiaire soluble dans le «sel fondu », conduit à des catalyseurs dont la sélectivité en isomères les plus ramifiés est importante et stable au cours du temps et dont l'activité est élevée.

L'objet de l'invention est un procédé de dimérisation sélective du propylène en dimères ramifiés dans lequel on utilise une composition catalytique comprenant au moins un composé du nickel mélangé ou complexé avec au moins une phosphine tertiaire portant un groupe fonctionnel, dissous au moins en partie dans un milieu non aqueux à caractère ionique (milieu de type « sels fondus »), résultant de la mise en contact d'au moins un halogénure d'aluminium (Produit B) avec au moins un halogénure d'ammonium quaternaire et/ou au moins un halogénure de phosphonium quaternaire (Produit A), le milieu de type « sels fondus » pouvant en outre comprendre au moins un composé organométallique d'aluminium (Produit C).

Ainsi, le milieu de type « sels fondus » dans lequel est dissous le composé de nickel mélangé ou complexé avec au moins une phosphine tertiaire portant un groupe fonctionnel ou au moins une phosphite portant un groupe fonctionnel est constitué par mélange :
a) d'au moins un halogénure, plus particulièrement chlorure et/ou bromure, d'ammonium quaternaire et/ou phosphonium quaternaire (Produit A) ;
b) d'au moins un halogénure d'aluminium (Produit B) ; et
c) éventuellement d'au moins un composé organométallique d'aluminium (Produit C).

Les halogénures d'ammonium et/ou de phosphonium quaternaires utilisables dans le cadre de l'invention (Produit A) répondent de préférence
- à l'une des formules générales NR¹R²R³R⁴X (à l'exception de NH₄X), PR¹R²R³R⁴X, R¹R²N=CR³R⁴X ou R¹R²P=CR³R⁴X, dans lesquelles X représente Cl ou Br et R¹, R², R3 et R⁴, identiques ou différents, représentent chacun l'hydrogène ou un reste hydrocarbyle de 1 à 12 atomes de carbone par exemple des groupements alkyles, saturés ou non saturés, cycloalkyles ou aromatiques, aryles ou aralkyles, comprenant de 1 à 12 atomes de carbone, étant entendu que, de préférence, un seul des substituants R¹, R², R³ et R⁴ représente l'hydrogène ;
- ou encore à l'une des formules générales : 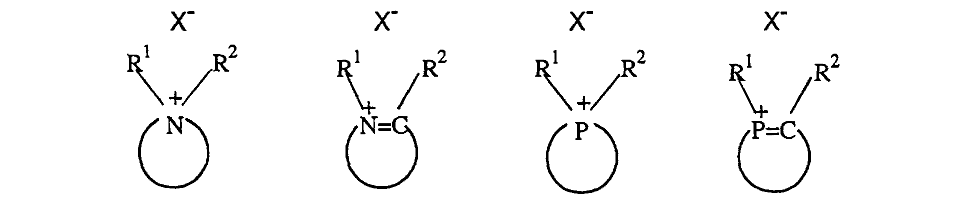
dans lesquelles les hétérocycles azotés ou phosphorés comportant 1, 2 ou 3 atomes d'azote et/ou de phosphore sont constitués de 4 à 10 atomes et X, R¹ et R² sont définis comme précédemment.

A titre d'exemples, on peut citer le chlorure de tétrabutylphosphonium, le chlorure de N-butylpyridinium, le bromure d'éthylpyridinium, le chlorure de butyl-3 méthyl-1 imidazolium, le chlorure de diéthylpyrazolium, le chlorhydrate de pyridinium, le chlorure de triméthylphénylammonium et le chlorure d'éthyl-3 méthyl-1 imidazolium. Ces sels peuvent être utilisés seuls ou en mélanges.

Les halogénures d'aluminium utilisés comme Produits B selon l'invention sont essentiellement le chlorure et le bromure d'aluminium.

Les composés organométalliques d'aluminium utilisés comme Produits C facultatifs selon l'invention, ont pour formule générale AlRₓX₃₋ₓ dans laquelle R est un reste alkyle, linéaire ou ramifié, comportant de 2 a 8 atomes de carbone, X étant le chlore ou le brome et x ayant une valeur égale à 1, 2 ou 3. A titre d'exemples, on peut utiliser le sesquichlorure d'isobutylaluminium, le sesquichlorure d'éthylaluminium, le dichloroisobutylaluminium, le dichloroéthylaluminium et le chlorodiéthylaluminium.

Les composants des « sels fondus » tels que définis ci-dessus sont en général mis en oeuvre dans des rapports molaires A:B de 1:0.5 à 1:3, de préférence de 1:1 à 1:2 ; le Produit C est mis en oeuvre dans un rapport molaire avec le Produit B au plus égal à 100:1 et de préférence de 0.005:1 à 10:1. Il est néanmoins nécessaire que les composants et leurs proportions soient tels que le mélange soit liquide à la température à laquelle se fait l'introduction du composé du nickel et de la phosphine tertiaire fonctionnalisée, bien que la réaction catalytique de dimérisation puisse se faire à une température inférieure ou supérieure à la température de fusion de la composition catalytique.

Les composés du nickel utilisés dans les compositions catalytiques de l'invention sont par exemple le chlorure, le bromure, le sulfate, les carboxylates, (par exemple l'éthyl-2 hexanoate), les phénates et l'acétylacétonate. On peut également utiliser les complexes organométalliques du nickel contenant ou non des phosphines. Ces complexes de nickel sont utilisés en mélange avec une phosphine tertiaire fonctionnalisée. On peut également utiliser des complexes du nickel déjà complexés avec une phosphine tertiaire portant une fonction.

Les phosphines fonctionnelles utilisées en mélange avec (ou pour complexer) les composés de nickel selon l'invention répondent aux formules générales PR'₁R'₂R'₃ et R'₁R'₂P-R'-PR'₁R'₂ dans lesquelles R'₁, R'₂ et R'₃, identiques ou différents, sont des radicaux alkyles, cycloalkyles, aryles ou aralkyles comportant de 1 à 10 atomes de carbone dont l'un au moins porte un groupe fonctionnel tel qu'une amine, une amine cyclique, un hétérocycle azoté, un ester, un acide, un alcool, un ammonium quaternaire, un phosphonium quaternaire, un sulfonium, un sulfonate ou un phosphonate et R' est un reste bivalent aliphatique de 1 à 6 atomes de carbone.

Les phosphines fonctionnelles peuvent être choisies parmi les composés contenant des substituants pyridine ou imidazole, ou leurs dérivés de quaternisation à substituants pyridinium ou imidazolium, qui répondent aux formules 1 à 7 présentées plus loin.

Comme phosphines fonctionnelles portant un substituant pyridine, on peut utiliser la dicyclopentyl-phosphinoéthyl-2-pyridine-4, de formule (1), la dicyclopentyl-phosphinoéthyl-2-pyridine-2, de formule (2), la diisobutyl-phosphinoéthyl-2-pyridine-4, de formule (1b), la diisopropyl-phosphinoéthyl-2 pyridine-4, de formule (4), et leurs dérivés de quaternisation de formule (3), dans laquelle R est un groupe alkyle comportant 1 à 10 atomes de carbone et X est un anion faiblement coordinant. A titre d'exemples d'anions faiblement coordinants, on peut citer le tétrafluoroborate, l'hexafluorophosphate, le tétrachloroaluminate, l'hexafluoroantimonate, les anions carboxylates tels que l'acétate et le trifluoroacétate, le trifluorosulfonate, ainsi que les anions N(CF₃SO₂)₂⁻ et C(CF₃SO₂)₃⁻. Les dérivés de quaternisation sont par exemple le tétrafluoroborate de dicyclopentylphosphinoéthyl-2 N-éthylpyridinium, de formule (3a), ou le chlorure de dicyclopentyl-phosphinoéthyl-2 N-éthylpyridinium, de formule (3b).

Comme phosphines fonctionnelles portant un substituant imidazole, on peut utiliser par exemple la dicyclopentyl-phosphinoéthyl-2 N-imidazole, de formule (5), la diisopropyl-phosphinoéthyl-2 N-imidazole de formule (7), la diisobutyl-phosphinoéthyl-2 N-imidazole de formule (7b) et leurs dérivés de quaternisation de formule (6), dans laquelle R est un groupe alkyle comportant 1 à 10 atomes de carbone et X est un anion faiblement coordinant (comme défini ci-dessus), tels que le tétrafluoroborate de dicyclopentyl-phosphinoéthyl-2 méthyl-1 imidazolium de formule (6a).

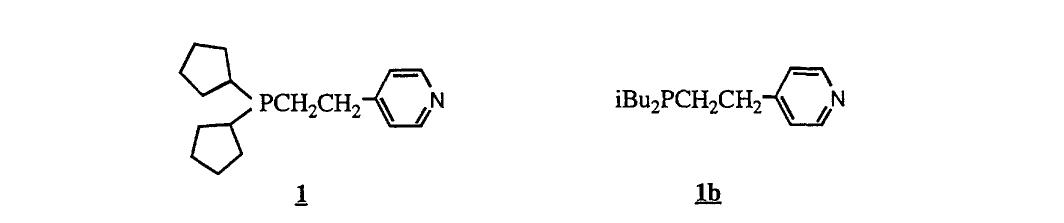

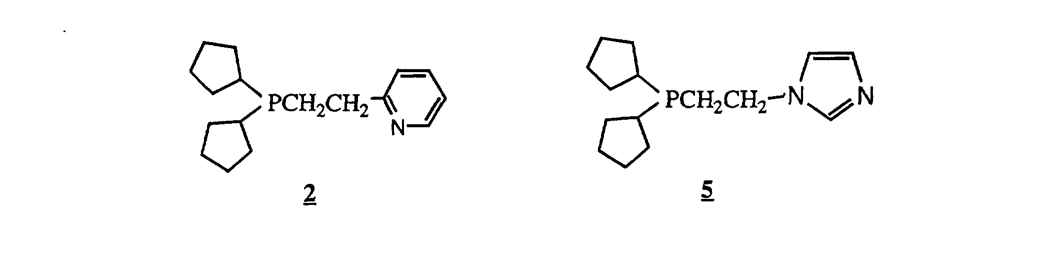

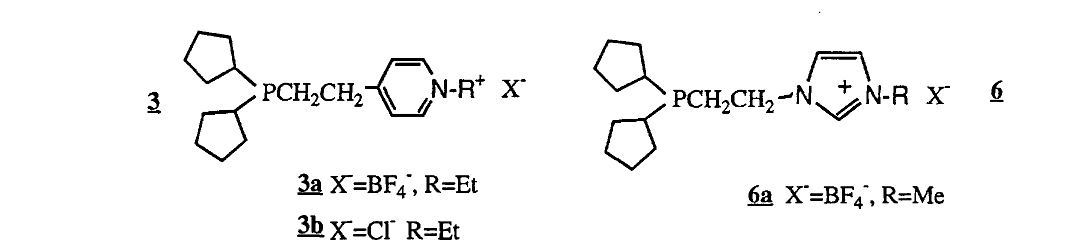

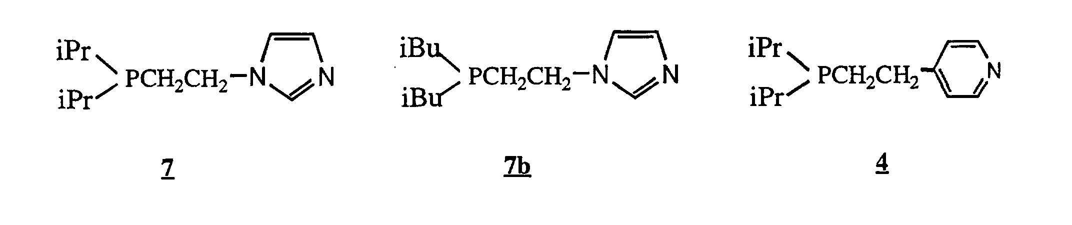

A titre d'exemples de composés du nickel utilisables dans la constitution des compositions catalytiques de l'invention, on peut citer les complexes [NiCl₂,1.5P (dicyclopentyléthyl-2 pyridine-4)]₂, [NiCl₂,2P (dicyclopentyléthyl-2 N éthylpyridinium tétrafluoroborate)]₂ [Ni₂Cl₄, (dicyclopentylphosphinoéthyl-2 N-éthylpyridinium tétrafluoroborate)₃, 1,5 CH₂Cl₂], NiCl₂,2 pyridine en mélange avec au moins un équivalent de phosphine tertiaire fonctionnalisée, le chlorure de nickel en mélange avec au moins un équivalent de dicyclopentylphosphinoéthyl-2 pyridine-4, l'acétate de nickel en mélange avec au moins un équivalent de dicyclopentylphosphinoéthyl-2 pyridine-4, l'octoate (éthyl-2 hexanoate) de nickel en mélange avec au moins un équivalent de dicyclopentylphosphinoéthyl-2 pyridine-4 et le chlorure de π-allyl-nickel dicyclopentylphosphinoéthyl-2 pyridine-4.

Les composés entrant dans la composition catalytique selon l'invention peuvent être mélangés dans un ordre quelconque. Le mélange peut se faire par une simple mise en contact suivie d'une agitation jusqu'à formation d'un liquide homogène. Ce mélange peut être fait en dehors du réacteur de dimérisation ou d'oligomérisation ou de préférence dans ce réacteur.

Le propylène soumis à la dimérisation sélective de l'invention peut être utilisé pur ou dilué par un alcane tel qu'on le trouve dans les « coupes » C₃ issues des procédés de raffinage du pétrole, comme le craquage catalytique ou le craquage a la vapeur.

La réaction catalytique de dimérisation du propylène mise en oeuvre dans le procédé de l'invention peut être conduite en système fermé, en système semi-ouvert ou en continu, avec un ou plusieurs étages de réaction. Une vigoureuse agitation doit assurer un bon contact entre le ou les réactifs et le mélange catalytique. La température de réaction peut être de -40 à +70 °C, de préférence de -20 à +50 °C. On peut opérer au-dessus ou en dessous de la température de fusion du milieu, l'état solide dispersé n'étant pas une limitation au bon déroulement de la réaction. La chaleur engendrée par la réaction peut être éliminée par tous les moyens connus de l'homme du métier. La pression peut aller de la pression atmosphérique à 20 MPa, de préférence de la pression atmosphérique à 5 MPa. Les produits de la réaction et le ou les réactifs qui n'ont pas réagi sont séparés du système catalytique par simple décantation, puis fractionnés.

Les exemples suivants illustrent l'invention sans en limiter la portée.

### EXEMPLE 1 : Préparation du solvant ionique

On a mélangé à température ambiante 17,5 g (0,1 mole) de chlorure de butyl-1 méthyl-3 imidazolium, 16,3 g (0,122 mole) de chlorure d'aluminium sublimé, 1,6 g (0,0126 mole) de dichloroéthylaluminium. On a obtenu ainsi un liquide.

### EXEMPLE 2 :

### Préparation du complexe [NiCl₂,1.5P (dicyclopentyléthyl-2 pyridine-4)]₂.

Dans un tube de Schlenk maintenu sous atmosphère d'argon, on introduit 2,37 g de NiCl₂•6H₂O et 10 ml de méthanol absolu. Après dissolution du sel de nickel, on ajoute 20 ml de pentane. On agite les 2 phases et on ajoute, 5,33 g de la phosphine tertiaire de formule (1) (20 mmoles). Après 2 heures d'agitation, on filtre l'insoluble rouge. On obtient 5,82g. L'analyse élémentaire correspond au complexe de formule NiCl₂•1,5 P dicyclopentyléthyl-2 pyridine-4)]₂ (M = 1085 g ; 10,7 % en poids de Ni).

### EXEMPLE 3 : Quaternisation de la pyridine du complexe décrit dans l'Exemple 2.

Dans un tube de Schlenk, on place 3,72 g du complexe décrit dans l'Exemple 2 et on ajoute du dichlorométhane. On ajoute ensuite, goutte à goutte, une solution d'oxonium de tétrafluoroborate dans le dichlorométhane (2,14 g de Et₃O+BF₄⁻). On laisse agiter 4 heures, temps au bout duquel on obtient une solution rouge. On évapore le solvant et on ajoute 20 ml d'éther. On filtre le solide cristallin rouge obtenu. On obtient 4,56g. L'analyse élémentaire correspond au complexe de formule : Ni₂Cl₄(P-N⁺Et BF₄⁻)₃, 1,5CH₂Cl₂, où P-N est le ligand de formule (1).

### EXEMPLE 4 : Dimérisation du propylène

Un réacteur en verre, muni d'une sonde de mesure de température, d'un barreau aimanté dans l'étage du bas (de volume 20 ml) pour assurer une bonne agitation et d'une double enveloppe permettant la circulation d'un liquide de réfrigération, a été purgé d'air et d'humidité et maintenu à la pression atmosphérique de propylène à 99 % de pureté. On y a introduit 0,03 mmole du complexe préparé dans l'Exemple 2 (0,06 mmole de Ni) puis on a abaissé la température à 10 °C et injecté à l'aide dune seringue 5 ml de la composition liquide préparée ci-dessus (Exemple 1) et 7 ml d'heptane. On a mis l'agitation en route et on a observé immédiatement une absorption de propylène. Quand l'étage supérieur non agité a été plein de liquide, on a soutiré la plus grande partie de la phase hydrocarbonée. On a arrêté la réaction au bout de 7 heures (5 soutirages). A ce moment, on avait produit 175 kg de produits par gramme de Ni. L'analyse des différentes fractions a montré qu'elles étaient composées à 77 % de dimères. La composition des dimères qui était pratiquement identique dans toutes les fractions comportait 67 % de diméthyl-2,3 butènes, et 29 % de méthyl-pentènes le reste étant des n-hexènes.

### EXEMPLE 5: Dimérisation du propylène

On a opéré comme dans l'Exemple 4 à ceci près qu'on a utilisé le sel fondu préparé à cet effet, et qu'on a introduit 0,05 mmole d'octoate (éthyl-2 hexanoate) de nickel et de 0,5 mmole de la dicyclopentylphosphinoéthyl-2 pyridine-4. La réaction a duré 7 heures et 15 minutes, temps au bout duquel on a soutiré 5 fractions et produit 220 kg de produits par gramme de Ni. La sélectivité en dimères est de 78 %. La sélectivité en diméthyl-2,3-butènes est de 66 % dans la première fraction et de 63 % dans la dernière.

### EXEMPLE 6: Dimérisation du propylène

On a opéré comme dans l'Exemple 4 à ceci près qu'on a utilisé le sel fondu préparé à cet effet, et qu'on a introduit 45 mg du complexe préparé dans l'Exemple 3. La réaction a duré 7 heures et 15 minutes, temps au bout duquel on a soutiré 5 fractions et produit 117 kg de produits par gramme de Ni. La sélectivité en dimères est de 74-79 %. La sélectivité en diméthyl-2,3-butènes est de 65 % et elle reste constante dans les différentes fractions.

### EXEMPLE 7 (comparatif) : Dimérisation du propylène

On a opéré comme dans l'Exemple 4 à ceci près qu'on a utilisé le sel fondu préparé comme dans l'Exemple 1 mais en introduisant 0,05 mmole de complexe NiCl₂·2P (cyclohexyl)₃. On a laissé la réaction pendant 8 heures et 30 minutes, temps au bout duquel on a soutiré 10 fractions. On a produit 137 kg de produits par gramme de Ni, avec une sélectivité de 83 % en dimères. La sélectivité en diméthyl-2,3 butènes est de 70 % dans la première fraction ; elle chute à 35 % dans la troisième et à 10 % dans la sixième. Elle est de 6 % dans la dixième.

## Revendications

1. Procédé de dimérisation sélective du propylène principalement en dimères ramifiés **caractérisé en ce que** l'on met le propylène en contact avec une composition catalytique comprenant au moins un composé du nickel mélangé ou complexé avec au moins une phosphine tertiaire portant un groupe fonctionnel dissous au moins en partie dans un milieu non aqueux à caractère ionique résultant de la mise en contact d'au moins un halogénure d'aluminium (Produit B) avec au moins un halogénure d'ammonium quaternaire et/ou au moins un halogénure de phosphonium quaternaire (Produit A).

2. Procédé selon la revendication 1 **caractérisé en ce que** le composé du nickel est choisi parmi le chlorure, le bromure, le sulfate, les carboxylates, les phénates et l'acétylacétonate.

3. Procédé selon la revendication 1 ou 2 **caractérisé en ce que** la phosphine tertiaire portant un groupe fonctionnel répond à l'une des formules générales PR'₁R'₂R'₃ ou R'₁R'₂P-R'-PR'₁R'₂, dans lesquelles R'₁, R'₂ et R'₃, identiques ou différents, sont des radicaux alkyles, cycloalkyles, aryles ou aralkyles comportant de 1 à 10 atomes de carbone dont l'un au moins porte un groupe fonctionnel choisi parmi les groupes amines, amines cycliques, hétérocycles azotés, esters, acides, alcools, ammoniums quaternaires, phosphoniums quaternaires, sulfoniums, sulfonates et phosphonates et R' est un reste bivalent aliphatique de 1 à 6 atomes de carbone.

4. Procédé selon la revendication 3 **caractérisé en ce que** la phosphine tertiaire fonctionnelle est choisie parmi les phosphines contenant des substituants pyridine ou imidazole et leurs dérivés de quaternisation à substituants pyridinium ou imidazolium.

5. Procédé selon la revendication 4 **caractérisé en ce que** la phosphine tertiaire fonctionnelle portant un substituant pyridine ou imidazole est choisie parmi la dicyclopentyl-phosphinoéthyl-2-pyridine-4, la dicyclopentyl-phosphinoéthyl-2-pyridine-2, la diisobutyl-phosphinoéthyl-2-pyridine-4, la diisopropyl-phosphinoéthyl-2 pyridine-4, la dicyclopentyl-phosphinoéthyl-2 N-imidazole, la diisopropyl-phosphinoéthyl-2 N-imidazole et la diisobutyl-phosphinoéthyl-2 N-imidazole.

6. Procédé selon la revendication 4 **caractérisé en ce que** la phosphine tertiaire fonctionnelle portant un substituant pyridinium ou imidazolium est un dérivé de quaternisation répondant à l'une des formules (3) et (6) 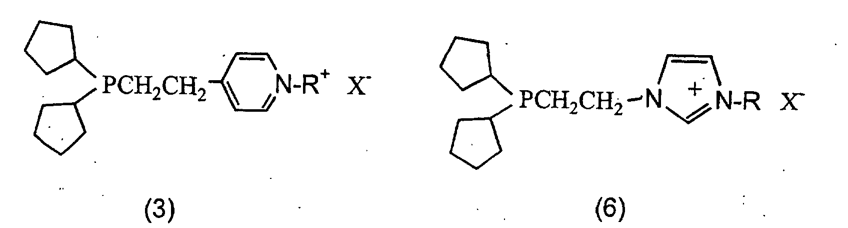 dans lesquelles R est un groupe alkyle comportant 1 à 10 atomes de carbone et X est un anion faiblement coordinant.

7. Procédé selon la revendication 6 **caractérisé en ce que** l'anion faiblement coordinant est choisi parmi le tétrafluoroborate, l'hexafluorophosphate, le tétrachloroaluminate, l'hexafluoroantimonate, les anions carboxylates tels que l'acétate et le trifluoroacétate, le trifluorosulfonate, et les anions N(CF₃SO₂)₂⁻ et C(CF₃SO₂)₃⁻.

8. Procédé selon la revendication 6 ou 7 **caractérisé en ce que** la phosphine tertiaire fonctionnelle portant un substituant pyridinium ou imidazolium est choisie parmi le tétrafluoroborate de dicyclopentyl-phosphinoéthyl-2 N-éthylpyridinium, le chlorure de dicyclopentyl-phosphinoéthyl-2 N-éthylpyridinium et le tétrafluoroborate de dicyclopentyl-phosphinoéthyl-2 méthyl-1 imidazolium.

9. Procédé selon l'une des revendications 1 à 8 **caractérisé en ce que** le composé du nickel mélangé ou complexé avec au moins une phosphine tertiaire portant un groupe fonctionnel est choisi parmi les complexes NiCl₂•1,5 P (dicyclopentyléthyl-2 pyridine-4)]₂, [NiCl₂·2P (dicyclopentyléthyl-2 N éthylpyridinium tétrafluoroborate)]2 [Ni₂Cl₄, (dicyclopentylphosphinoéthyl-2 N-éthylpyridinium tétrafluoroborate)₃, 1,5CH₂Cl₂], NiCl₂,2 pyridine en mélange avec au moins un équivalent de phosphine tertiaire fonctionnalisée, le chlorure de nickel en mélange avec au moins un équivalent de dicyclopentylphosphinoéthyl-2 pyridine-4, l'acétate de nickel en mélange avec au moins un équivalent de dicyclopentylphosphinoéthyl-2 pyridine-4, l'octoate (éthyl-2 hexanoate) de nickel en mélange avec au moins un équivalent de dicyclopentylphosphinoéthyl-2 pyridine-4 et le chlorure de π-allyl-nickel dicyclopentylphosphinoéthyl-2 pyridine-4.

10. Procédé selon l'une des revendications 1 à 9 **caractérisée en ce que** l'halogénure d'ammonium quaternaire ou l'halogénure de phosphonium quaternaire utilisable comme Produit A répond à l'une des formules générales NR¹R²R³R⁴X, à l'exception de NH4X, PR¹R²R³R⁴X, R¹R²N=CR³R⁴X ou R¹R²P=CR³R⁴X, dans lesquelles X représente Cl ou Br et R¹, R², R³ et R⁴, identiques ou différents, représentent chacun l'hydrogène ou un reste hydrocarbyle de 1 à 12 atomes de carbone, ou encore à l'une des formules générales : 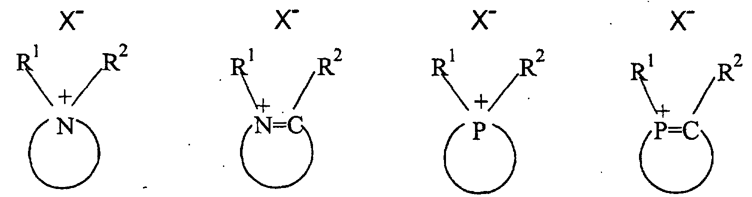 dans lesquelles les hétérocycles azotés ou phosphorés comportant 1, 2 ou 3 atomes d'azote et/ou de phosphore sont constitués de 4 à 10 atomes et X, R1 et R2 sont définis comme précédemment.

11. Procédé selon la revendication 10 **caractérisé en ce que** l'halogénure d'ammonium quaternaire ou l'halogénure de phosphonium quaternaire est le chlorure de tétrabutylphosphonium, le chlorure de N-butylpyridinium, le bromure d'éthylpyridinium, le chlorure de butyl-3 méthyl-1 imidazolium, le chlorure de diéthylpyrazolium, le chlorhydrate de pyridinium ou le chlorure de triméthylphénylammonium, le chlorure d'éthyl-3 méthyl-1 imidazolium.

12. Procédé selon l'une des revendications 1 à 11 **caractérisé en ce que** l'halogénure d'aluminium utilisé comme Produit B est le chlorure ou le bromure d'aluminium.

13. Procédé selon l'une des revendications 1 à 12 **caractérisé en ce que** les Produits A et B sont mis en oeuvre dans un rapport molaire A:B de 1:0.5 à 1:3.

14. Procédé selon l'une des revendications 1 à 13 **caractérisé en ce que** le milieu non aqueux à caractère ionique comprend en outre un Produit C, consistant en au moins un composé organométallique de l'aluminium.

15. Procédé selon la revendication 14 **caractérisé en ce que** le composé organométallique de l'aluminium utilisé comme Produit C répond à la formule générale AlRₓX₃₋ₓ, dans laquelle R est un reste alkyle, linéaire ou ramifié, comportant de 2 à 8 atomes de carbone, X étant le chlore ou le brome et x a une valeur égale à 1, 2 ou 3.

16. Procédé selon la revendication 14 ou 15 **caractérisé en ce que** le Produit C est le sesquichlorure d'isobutylaluminium, le sesquichlorure d'éthylaluminium, le dichloroisobutylaluminium, le dichloroéthylaluminium ou le chlorodiéthylaluminium.

17. Procédé selon l'une des revendications 14 à 16 **caractérisé en ce que** le Produit C est mis en oeuvre dans un rapport molaire avec le Produit B au plus égal à 100:1

18. Procédé selon l'une des revendications 1 à 17 **caractérisé en ce que** la réaction est conduite en système fermé, en système semi-ouvert ou en continu, avec un ou plusieurs étages de réaction, sous agitation et à une température de -40 à +70 °C.

19. Procédé selon l'une des revendications 1 à 18 **caractérisé en ce que** le propylène est contenu dans une « coupe » C₃ issue d'un procédé de raffinage du pétrole.

## Patentansprüche

1. Verfahren zur selektiven Dimerisierung von Propylen hauptsächlich in verzweigte Dimere, **dadurch gekennzeichnet, dass** man das Propylen in direkten Kontakt mit einer katalytischen Zusammensetzung bringt, die eine mit wenigstens einem tertiären Phosphin vermischte oder komplexierte Nickelverbindung mit einer wenigstens teilweise in einem nicht-wässrigen Medium mit ionischer Eigenschaft gelösten funktionellen Gruppe, welche aus dem Kontaktieren von wenigstens einem Aluminiumhalogenid (Produkt B) mit wenigstens einem quaternären Ammoniumhalogenid und/oder wenigstens einem quaternären Phosphoniumhalogenid (Produkt A) herrührt, enthält.

2. Verfahren nach Anspruch 1, **dadurch gekennzeichnet, dass** die Nickelverbindung aus Chlorid, Bromid, Sulfat, Carboxylaten, Phenaten und Acetylacetonaten gewählt ist.

3. Verfahren nach Anspruch 1 oder 2, **dadurch gekennzeichnet, dass** das tertiäre Phosphin eine funktionellen Gruppe der allgemeinen Formeln PR'₁R'₂R'₃ oder R'₁R'₂P- R'-PR'₁R'₂ trägt, in welchen R'₁, R'₂ und R'₃ - identisch oder unterschiedlich - Alkyl-, Cycloalkyl-, Aryl- oder Aralkylradikale mit von 1 bis 10 Kohlenstoffatomen sind, von denen wenigstens eines eine funktionelle Gruppe aus' der Gruppe der Amine, der cyclischen Amine, der stickstoffhaltigen Heterozyklen, der Ester, der Säuren, der Alkohole, des quaternären Ammoniums, des quaternären Phosphoniums, Sulfoniums, Sulfonate und Phosphonate aufweist und R' ein zweiwertiger aliphatischer Rest aus 1 bis 6 Kohlenstoffatomen ist.

4. Verfahren nach Anspruch 3, **dadurch gekennzeichnet, dass** das funktionelle tertiäre Phosphin aus den Phosphinen gewählt ist, welche Pyridin- oder Imidazolsubstituenten und deren Derivate der Quaternisierung der Pyridinium- oder Imidazoliumsubstitutenten enthalten.

5. Verfahren nach Anspruch 4, **dadurch gekennzeichnet, dass** das funktionelle, einen Pyridin- oder Imidazolsubstituenten tragende, tertiäre Phosphin ausgewählt ist aus 4-(2-Dicyclopentylphosphinoethyl)-Pyridin, 2-(2-Dicyclopentylphosphinoethyl)-Pyridin, 4-(2-Diisobutylphosphinoethyl)-Pyridin, 4-(2-Diisopropylphosphinoethyl)-Pyridin, N-(2-Dicyclopentylphosphinoethyl)-Imidazol, N-(2-Diisopropylphosphinoethyl)-Imidazol und N-(2-Diisobutylphosphinoethyl)-Imidazol.

6. Verfahren nach Anspruch 4, **dadurch gekennzeichnet, dass** das funktionale, einen Pyridinium- oder Imidazoliumsubstituenten tragende, tertiäre Phosphin ein Derivat der Quaternisierung entsprechend einer der Formeln (3) und (6) 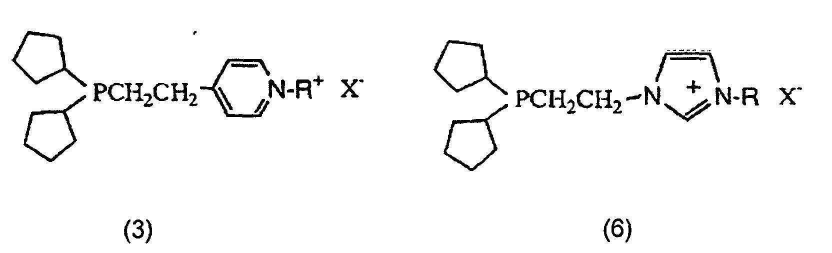 ist, in welchem R eine 1 bis 10 Kohlenstoffatome umfassende Alkylgruppe ist und X ein schwach bindendes Anion ist.

7. Verfahren nach Anspruch 6, **dadurch gekennzeichnet, dass** das schwach koordinierende Anion aus Tetrafluoroborat, Hexafluorophosphat, Tetrachloroaluminat, Hexafluoroantimonat, Carboxylatanionen wie zum Beispiel Acetat und Trifluoroacetat, Trifluorosulfonat, und den Anionen N(CF₃SO₂)₂⁻ und C(CF₃SO₂)₃⁻ gewählt ist.

8. Verfahren nach Anspruch 6 oder 7, **dadurch gekennzeichnet, dass** das funktionale tertiäre Phosphin mit einem Pyridinium- oder Imidazoliumsubstituent aus Tetrafluoroborat von (2-Dicyclopentylphosphinoethyl)-N-Ethylpyridinium, (2-Dicyclopentylphosphinoethyl)-N-Ethylpyridiniumchlorid und dem Tetrafluoroborat von (2-Dicyclopentylphosphinoethyl)-1-Methylimidazolium ausgewählt ist.

9. Verfahren nach einem der Ansprüche 1 bis 8, **dadurch gekennzeichnet, dass** die mit wenigstens einem tertiären Phosphin vermischte oder komplexierte Nickelverbindung mit einer funktionellen Gruppe aus den Komplexen [NiCl₂·1,5P(4-(2-Dicyclopentylethyl)-Pyridin)]₂, [NiCl₂·2P(2-Dicyclopentyl-phosphinoethyl)-N-Ethylpyridiniumtetrafluoroborat)]₂, [Ni₂Cl₄, ((2Dicyclopentylphosphinoethyl)-N-Ethylpyridiniumtetrafluoroborat)₃, 1,5CH₂Cl₂], NiCl₂·2 Pyridin, vermischt mit wenigstens einem Äquivalent von funktionalisiertem tertiären Phosphin, Nickelchlorid vermischt mit wenigstens einem Äquivalent von 4-(2-Dicyclopentylphosphinoethyl)-Pyridin, Nickelacetat vermischt mit wenigstens einem Äquivalent von 4-(2-Dicyclopentylphosphinoethyl)-Pyridin, Nickel-(2-Ethylhexanoat, Oktoat) vermischt mit wenigstens einem Äquivalent von 4-(2-Dicyclopentylphosphinoethyl)-Pyridin und Chlorid von π-Allyl-Nickel 4-(2-Dicyclopentylphosphinoethyl)-Pyridin gewählt ist.

10. Verfahren nach einem der Ansprüche 1 bis 9, **dadurch gekennzeichnet, dass** die als Produkt A verwendbaren quaternären Ammoniumhalogenide oder quarternären Phosphoniumhalogenide einer der allgemeinen Formeln NR¹R²R³R⁴X entspricht, mit Ausnahme von NH₄X, PR¹R²R³R⁴X, R¹R²N=CR³R⁴X oder R¹R²P=CR³R⁴X, in denen X für Cl oder Br steht und R¹, R², R³ und R⁴ - identisch oder unterschiedlich - jeweils Wasserstoff oder einen Kohlenwasserstoffrest von 1 bis 12 Kohlenstoffatomen darstellen, oder auch einer der allgemeinen Formeln: 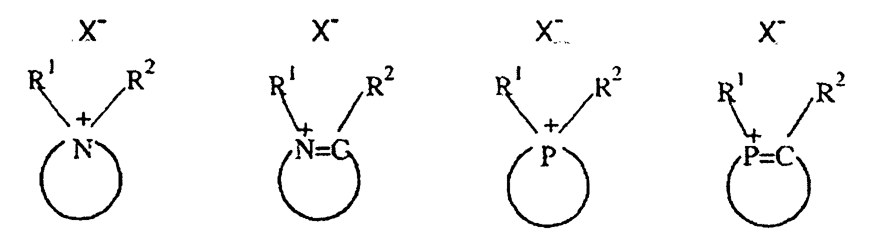 entspricht, in denen die stickstoff- oder phosphorhaltigen Heterozyklen, umfassend 1, 2 oder 3 Stickstoff- und/oder Phosphoratome, aus 4 bis 10 Atomen aufgebaut sind und X, R1 und R2 wie vorher definiert sind.

11. Verfahren nach Anspruch 10, **dadurch gekennzeichnet, dass** das quaternäre Ammoniumhalogenid oder das quaternäre Phosphoniumhalogenid das Chlorid von Tetrabutylphosphonium, das Chlorid von N-Butylpyridinium, das Bromid von Ethylpyridinium, das Chlorid von 3-Butyl-1-Methyl-imidazolium, das Chlorid von Diethylpyrazolium, das Chlorhydrat von Pyridinium oder das Chlorid von Trimethylphenylammonium, das Chlorid von 3-Ethyl-1-Methyl-Imidazolin ist.

12. Verfahren nach einem der Ansprüche 1 bis 11, **dadurch gekennzeichnet, dass** das als Produkt B verwendete Aluminiumhalogenid ein Chlorid oder Bromid von Aluminium ist.

13. Verfahren nach einem der Ansprüche 1 bis 12, **dadurch gekennzeichnet, dass** die Produkte A und B im Molverhältnis A:B von 1:0.5 bis 1:3 eingesetzt werden.

14. Verfahren nach einem der Ansprüche 1 bis 13, **dadurch gekennzeichnet, dass** das nicht-wässrige Medium mit ionischer Eigenschaft zusätzlich ein Produkt C enthält, welches aus wenigstens einer organometallischen Aluminiumverbindung besteht.

15. Verfahren nach Anspruch 14, **dadurch gekennzeichnet, dass** die als Produkt C verwendete organometallische Aluminiumverbindung der allgemeinen Formel AlRₓX₃₋ₓ entspricht, in welcher R ein 2 bis 8 Kohlenstoffatome umfassender, linearer oder verzweigter Alkylrest ist, wobei X Chlor oder Brom ist, und wo x einen Wert von 1, 2 oder 3 hat.

16. Verfahren nach Anspruch 14 oder 15, **dadurch gekennzeichnet, dass** das Produkt C Isobutylaluminium-Sesquichlorid, Ethylaluminium-Sesquichlorid, Isobutylaluminiumdichlorid, Ethylaluminiumdichlorid oder Diethylaluminiumchlorid ist.

17. Verfahren nach einem der Ansprüche 14 bis 16, **dadurch gekennzeichnet, dass** das Produkt C in einem Molverhältnis zum Produkt B entsprechend höchstens 100:1 eingesetzt wird.

18. Verfahren nach einem der Ansprüche 1 bis 17, **dadurch gekennzeichnet, dass** die Reaktion im geschlossenen System, im halb-offenen System oder kontinuierlich mit einer oder mehreren Reaktionsstufen unter Rühren und bei einer Temperatur von -40°C bis +70°C durchgeführt wird.

19. Verfahren nach einem der Ansprüche 1 bis 18, **dadurch gekennzeichnet, dass** das Propylen in einem aus einem Rohölraffinierungsverfahren erhaltenen C₃-"Schnitt" enthalten ist.

## Claims

1. A process for selective dimerisation of propylene principally into branched dimers, **characterized in that** the propylene is brought into contact with a catalytic composition comprising at least one nickel compound mixed or complexed with at least one tertiary phosphine carrying a functional group, at least partly dissolved in a non aqueous medium with an ionic nature resulting from bringing at least one aluminium halide (product B) into contact with at least one quaternary ammonium halide and/or at least one quaternary phosphonium halide (product A).

2. A process according to claim 1 **characterized in that** the nickel compound is selected from the chloride, bromide, sulphate, carboxylates, phenates and acetylacetonate.

3. A process according to claim 1 or claim 2 **characterized in that** the tertiary phosphine carrying a functional group has one of the general formulae PR'₁R'₂R'₃ or R'₁R'₂P-R'-PR'₁R'₂, where R'₁, R'₂ and R'₃, which may be identical or different, are alkyl, cycloalkyl, aryl or aralkyl radicals containing 1 to 10 carbon atoms at least one of which carries a functional group selected from an amine, a cyclic amine, a nitrogen-containing heterocycle, an ester, an acid, an alcohol, a quaternary ammonium, a quaternary phosphonium, a sulphonium, a sulphonate and a phosphonate group and R' is a divalent aliphatic residue containing 1 to 6 carbon atoms.

4. A process according to claim 3 **characterized in that** the functional tertiary phosphine is selected from phosphines containing pyridine or imidazole substituents and their quaternisation derivatives with pyridinium or imidazolium substituents.

5. A process according to claim 4 **characterized in that** the functional tertiary phosphine carrying a pyridine or imidazole substituent is selected from 2-dicyclopentylphosphinoethyl-4-pyridine, 2-dicyclopentylphosphinoethyl-2-pyridine, 2-diisobutylphosphinoethyl-4-pyridine, 2-diisopropylphosphinoethyl-4-pyridine, 2-dicyclopentylphosphinoethyl-N-imidazole, 2-diisopropylphosphinoethyl-N-imidazole and 2-diisobutylphosphinoethyl-N-imidazole.

6. A process according to claim 4 **characterized in that** the functional tertiary phosphine carrying a pyridinium or imidazolium substituent is a quaternisation derivative with one of formulae (3) or (6) 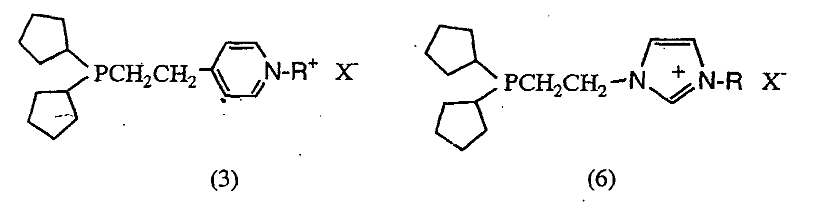 where R is an alkyl group containing 1 to 10 carbon atoms and X is a weakly co-ordinating anion.

7. A process according to claim 6 **characterized in that** the weakly co-ordinating anion is selected from tetrafluoroborate, hexafluorophosphate, tetrachloroaluminate, hexafluoroantimonate, carboxylate anions such as acetate or trifluoroacetate, trifluorosulphonate, and the N(CF₃SO₂)₂- and C(CF₃SO₂)₃⁻ anions.

8. A process according to claim 6 or claim 7 **characterized in that** the functional tertiary phosphine carrying a pyridinium or imidazolium substituent is selected from 2-dicyclopentylphosphinoethyl-N-ethyl pyridinium tetrafluoroborate, 2-dicyclopentylphosphinoethyl-N-ethyl pyridinium chloride and 2-dicyclopentylphosphinoethyl-1-methyl-imidazolium tetrafluoroborate.

9. A process according to any one of claims 1 to 8, **characterized in that** the nickel compound mixed or complexed with at least one tertiary phosphine carrying a functional group is selected from the following complexes: [NiCl₂. 1.5P(2-dicyclopentylethyl-4-pyridine)]₂; [NiCl₂. 2P(2-dicyclopentylethyl-N-ethyl pyridinium tetrafluoroborate)]; [Ni₂Cl₄, (2-dicyclopentylphosphinoethyl-N-ethyl pyridinium tetrafluoroborate)₃, 1.5 CH₂Cl₂]; NiCl₂, 2 pyridine mixed with at least one equivalent of functionalised tertiary phosphine; nickel chloride mixed with at least one equivalent of 2-dicyclopentylphosphinoethyl-4-pyridine; nickel acetate mixed with at least one equivalent of 2-dicyclopentylphosphinoethyl-4-pyridine; nickel (2-ethyl hexanoate) octoate mixed with at least one equivalent of 2-dicyclopentylphosphinoethyl-4-pyridine; and 2-dicyclopentylphosphinoethyl-4-pyridine π-allyl nickel chloride.

10. A process according to any one of claims 1 to 9, **characterized in that** the quaternary ammonium halide or quaternary phosphonium halide used as product A satisfies one of general formulae: NR¹R²R³R⁴X with the exception of NH₄X, PR¹R²R³R⁴X, R¹R²N=CR³R⁴X or R¹R²P=CR³R⁴X, where X represents Cl or Br and R¹, R², R³ and R⁴, which may be identical or different, each represent hydrogen or a hydrocarbyl residue containing I to 12 carbon atoms; or one of the following general formulae: 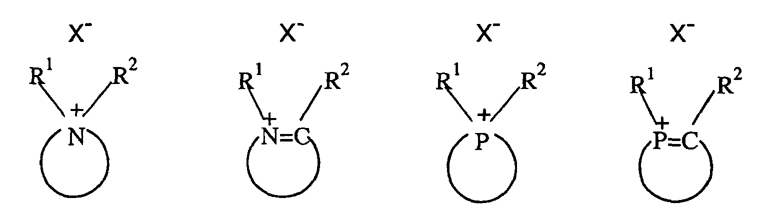 where the nitrogen-containing or phosphorus-containing heterocycles containing 1, 2 or 3 nitrogen and/or phosphorus atoms are constituted by 4 to 10 atoms and X, R¹ and R² are defined as above.

11. A process according to claim 10, **characterized in that** the quaternary ammonium halide or quaternary phosphonium halide is tetrabutyl phosphonium chloride, N-butyl pyridinium chloride, ethylpyridinium bromide, 3-butyl-1-methyl imidazolium chloride, diethylpyrazolium chloride, pyridinium hydrochloride, trimethylphenylammonium chloride, or 1-ethyl-3-methyl imidazolium chloride.

12. A process according to any one of claims 1 to 11, **characterized in that** the aluminium halide used as product B is aluminium chloride or bromide.

13. A process according to any one of claims 1 to 12, **characterized in that** products A and B are used in an A:B mole ratio of 1:0.5 to 1:3.

14. A process according to any one of claims 1 to 13, **characterized in that** the non aqueous medium with an ionic nature also comprises a product C, consisting of at least one organometallic aluminium compound.

15. A process according to claim 14, **characterized in that** the organometallic aluminium compound used as optional product C of the invention has general formula AlRₓX₃₋ₓ where R is a linear or branched alkyl residue containing 2 to 8 carbon atoms, X is chlorine or bromine and the value of x is 1, 2 or 3.

16. A process according to claim 14 or claim 15, **characterized in that** product C is isobutylaluminium sesquichloride, ethylaluminium sesquichloride, dichloroisobutylaluminium, dichloroethylaluminium, or chlorodiethylaluminium.

17. A process according to any one of claims 14 to 16, **characterized in that** product C is used in a mole ratio of at most 1:100 with product B.

18. A process according to any one of claims 1 to 17, **characterized in that** the reaction is carried out in a closed system, in a semi-open system or in a continuous system, with one or more reaction stages, with agitation and at a temperature of -40°C to +70°C.

19. A process according to any one of claims 1 to 18, **characterized in that** the propylene is contained in a C₃ cut from an oil refining process.
